# FASCICULE DE BREVET EUROPEEN

(11) **EP 1 231 902 B1**
(45) Date de publication et mention de la délivrance du brevet: **09.03.2005**
(21) Numéro de dépôt: 00979704.4
(22) Date de dépôt: 09.11.2000
(51) Int. Cl.: A61K 9/28

(54) **PROCEDE POUR LE PELLICULAGE DE COMPRIMES**
VERFAHREN ZUR BESCHICHTUNG VON TABLETTEN
FILM COATING METHOD FOR TABLETS

(30) Priorité: 10.11.1999 FR 9914104
(43) Date de publication de la demande: 21.08.2002
(73) Titulaire: Sanofi-Aventis, 75013 Paris (FR)
(72) Inventeur: BUISSON, Bertrand, F-04700 Oraison (FR); FRANCAIS, Eric, F-54134 Voinemont (FR); LANNE, Jean, Yves, F-33000 Bordeaux (FR); PERRUT, Michel, F-54000 Nancy (FR)
(74) Mandataire: Thouret-Lemaitre, Elisabeth
(86) Numéro de dépôt international: PCT/FR2000/003127
(87) Numéro de publication internationale: WO 2001/034122

(56) Documents cités:
- EP-A- 0 492 535
- EP-A- 0 706 821
- US-A- 5 178 325
- PHILLIPS E M ET AL: "RAPID EXPANSION FROM SUPERCRITICAL SOLUTIONS: APPLICATION TO PHARMACEUTICAL PROCESSES" INTERNATIONAL JOURNAL OF PHARMACEUTICS,NL,AMSTERDAM, vol. 94, 1993, pages 1-10, XP000675504 ISSN: 0378-5173

## Description

La présente invention se rapporte, d'une manière générale, à un procédé pour le pelliculage de comprimés.

En particulier, l'invention concerne un procédé pour le pelliculage de comprimés à l'aide d'un solvant porté à pression supercritique, c'est-à-dire un fluide en état supercritique, ou à l'aide d'un liquide subcritique et ce, en vue d'obtenir des comprimés faciles à conserver et à utiliser , en particulier à des fins thérapeutiques.

Depuis de nombreuses années, on assure au moyen d'un recouvrement pelliculaire, la protection des comprimés destinés à diverses utilisations et particulièrement des utilisations thérapeutiques en pharmacie humaine ou vétérinaire. Il s'agit en fait d'un recouvrement capable d'éviter le délitement de ces comprimés tout en limitant leur absorption en humidité de l'air qui les altérerait irréversiblement et en empêcherait une bonne conservation dans les conditions domestiques habituelles. Certains de ces revêtements ont également un rôle important sur la vitesse d'assimilation du ou des principe(s) actif(s) dans l'organisme. Ce pelliculage fait appel à des mélanges de composés bien connus, tels que des dérivés de la cellulose, certains polymères ou co-polymères acryliques ou méthacryliques et le polyéthylèneglycol, ainsi que des agents minéraux tels que le talc ou le dioxyde de titane, et des colorants. Ces agents de pelliculage sont mis en suspension dans un liquide qui est soit l'eau lorsqu'il n'y a pas d'incompatibilité avec les composants des comprimés, et en particulier avec le ou les principe(s) actif(s) qui y sont contenus, soit un solvant organique tel que le dichlorométhane . Cette opération de pelliculage est généralement mise en oeuvre dans des cylindres tournants à axe horizontal au sein desquels les comprimés sont constamment remués et soumis à une pulvérisation très fine de la suspension ou de la solution du mélange des agents de pelliculage dans le solvant choisi.

Toutefois, les procédés ci-dessus pour le pelliculage de comprimés ne sont pas dénués d'inconvénients. Ils nécessitent en effet la manipulation de solvants organiques, en particulier des solvants halogénés et provoquent l'émission de vapeurs de solvants organiques potentiellement toxiques dont les traces demeurent également au sein des comprimés pelliculés.

Par ailleurs, les procédés de pelliculage par mise en oeuvre d'une solution ou d'une suspension aqueuse nécessitent une phase de séchage pouvant durer plus de 10 heures dans certains cas.

La recherche d'un procédé pour le pelliculage de comprimés, capable d'éviter les inconvénients des procédés antérieurs tout en lui assurant une mise en oeuvre dans des systèmes ou installations généralement utilisés dans les procédés classiques de pelliculage, reste d'un intérêt primordial.

On sait qu'un fluide en état supercritique, c'est-à-dire dans un état caractérisé soit par une pression et une température respectivement supérieures à la pression et à la température critiques dans le cas d'un corps pur, soit par un point représentatif (pression, température) situé au-delà de l'enveloppe des points critiques représentés sur un diagramme (pression, température) dans le cas d'un mélange, présente, pour de très nombreuses substances, un pouvoir solvant élevé sans commune mesure avec celui observé dans ce même fluide à l'état de gaz comprimé. Il en est de même des liquides dits « subcritiques » c'est-à-dire dans un état caractérisé soit par une pression supérieure à la pression critique et par une température inférieure à la température critique dans le cas d'un corps pur, soit par une pression supérieure aux pressions critiques et une température inférieure aux températures critiques des composants dans le cas d'un mélange (Michel PERRUT, les Techniques de l'Ingénieur « Extraction par fluide supercritique », J2 770 - 1 à 12, 1999).

Les variations importantes et modulables du pouvoir solvant de ces fluides sont d'ailleurs utilisées dans de nombreux procédés d'extraction (solide/fluide), de fractionnement (liquide/fluide), de chromatographie analytique ou préparative, de traitement des matériaux (céramiques, polymères, ...). Des réactions chimiques ou biochimiques sont également réalisées dans de tels solvants.

Depuis une dizaine d'années, on a entrepris de nombreux travaux en vue de réaliser l'atomisation de substances liquides ou solides en utilisant des procédés fondés sur les propriétés des fluides supercritiques, tels les procédés de recristallisation par effet antisolvant ou l'expansion rapide de solutions supercritiques, conduisant à l'élaboration de poudres de très fine granulométrie, de l'ordre de 0,1 à 10 micromètres, ou même de poudres complexes où le principe actif est dispersé au sein d'un excipient ou encapsulé dans un agent protecteur. De même, on a développé divers procédés de pulvérisation mettant en oeuvre un fluide supercritique, en particulier pour disperser des gouttelettes de peinture, d'adhésif ou de revêtement comme décrit dans les brevets US-5009367 ; US-5057342 ; US-5066522; US-5254260 et US-5197800 ainsi que dans les brevets EP-0 388 923, EP-0 388 915, EP-0 421 796 et EP-0 506 067. On peut ainsi réduire de façon importante, de l'ordre de 30 à 60 %, la teneur des peintures en solvants organiques, diminuant d'autant les émissions de composés organiques volatiles qui en résultent, sans que la qualité finale du revêtement, et en particulier son aspect, n'en soit altérée.

L'un des avantages principaux des procédés mettant en oeuvre les fluides à pression supercritique réside dans la facilité de réaliser la séparation entre le solvant (le fluide) et les extraits et solutés, ainsi qu'il a été décrit dans de nombreuses publications et, pour certains aspects importants de mise en oeuvre, dans le brevet français FR-2 592 488. Les propriétés intéressantes de ces fluides sont d'ailleurs utilisées depuis longtemps en extraction solide-fluide et fractionnement liquide-fluide, ainsi qu'il est décrit dans l'article cité ci-dessus. En outre, il est également intéressant de noter qu'un solvant à pression supercritique a non seulement la propriété de dissoudre certains composés, propriété utilisée dans les procédés qui viennent d'être cités, mais également de se dissoudre de façon très importante dans les liquides et certains solides comme les polymères. Cette faculté est utilisée dans certains procédés de pulvérisation, d'imprégnation ou d'élaboration de mousses de polymères. Ainsi, il est connu que le dioxyde de carbone maintenu à pression supercritique et à une température voisine de sa température critique, peut se dissoudre dans les polymères thermoplastiques courants à raison de 10 à 30 % en masse selon la nature du polymère, entraînant d'ailleurs un gonflement important du polymère et un profond changement de ses propriétés physiques, avec une détérioration importante de ses propriétés mécaniques et un abaissement de sa température de transition vitreuse pouvant atteindre 40°C dans certains cas. Il est également connu que la décompression brutale de cette solution de fluide à pression supercritique dans un solide ou un liquide peut être réalisée dans une buse d'atomisation et ainsi conduire à l'élaboration de particules solides comme il a été décrit en particulier dans la demande de brevet WO-95121688 et dans différentes publications citant par exemple des résultats de pulvérisation de polyéthylène glycol (Weidner E., Steiner R., Knez.Z., dans « High Pressure Chemical Engineering », Elsevier 1996, ISBN 0-444-82475-8, p.223-228).

Les propriétés physico-chimiques du dioxyde de carbone ainsi que ses coordonnées critiques (pression critique : 7,4 MPa et température critique : 31°C) en font le fluide supercritique de choix utilisé comme solvant préféré dans de nombreuses applications d'autant plus qu'il ne présente pas de toxicité et est disponible à très bas prix et en très grande quantité. Solvant non polaire, le dioxyde de carbone porté à pression supercritique est parfois additionné également d'un co-solvant consistant en un solvant organique capable de modifier le pouvoir solvant de ce dioxyde de façon notable surtout vis-à-vis de molécules présentant une certaine polarité.

Or, il a maintenant été trouvé de manière surprenante qu'il est possible de procéder au pelliculage de comprimés à partir d'une suspension d'un agent de pelliculage au sein d'un fluide à pression supercritique que l'on pulvérise sur les comprimés à pelliculer.

En conséquence, l'invention se rapporte à un procédé pour le pelliculage de comprimés, procédé selon lequel on pulvérise sur les comprimés en question une suspension de l'agent de pelliculage dans un fluide à pression supercritique.

Ainsi, le procédé de l'invention se caractérise par l'utilisation des propriétés particulières des fluides à pression supercritique vis-à-vis des polymères et des liquides au sein desquels il se dissolvent de façon importante, ce qui selon l'invention va permettre la réalisation aisée d'une suspension très homogène de l'agent de pelliculage au sein du fluide, et sur l'utilisation de ces fluides à pression supercritique comme agent de pulvérisation à travers une buse, ou tout autre dispositif permettant de créer une très forte perte de charge, et d'assurer ainsi la pulvérisation de la suspension au sein de laquelle ils sont dissous.

Le procédé de l'invention peut être mis en oeuvre en réalisant d'abord, par agitation mécanique, une suspension très homogène d'un agent de pelliculage au sein d'un fluide à pression supercritique, généralement du dioxyde de carbone, puis en pulvérisant cette suspension en un fin brouillard de gouttelettes d'où le fluide sera immédiatement évacué lorsque l'opération est réalisée dans des conditions de température et de pression adéquates, c'est-à-dire à une température comprise entre la température ambiante et 60°C et à une pression comprise entre la pression atmosphérique et 200 bars. Lorsque le procédé de l'invention est mis en oeuvre sous pression, on opère généralement entre 30 et 200 bars de préférence à une pression comprise entre 100 et 200 bars et généralement à une température comprise entre 30 et 60°C. Préférentiellement, on pratique l'opération de pelliculage dans des conditions normales de température et de pression c'est-à-dire à température ambiante et à pression atmosphérique.

Cette pellicule sera d'autant mieux répartie sur les comprimés que ces derniers seront soumis à une agitation continue ainsi qu'il est déjà réalisé selon les techniques de pelliculage classiques au sein de tambours tournant autour de leur axe horizontal.

Par « agent de pelliculage », on entend tout composé généralement utilisé à cet effet, c'est-à-dire un composé capable, par recouvrement du comprimé, de former une pellicule ou enrobage, ou encore un mélange de tels composés. Cette pellicule peut avoir notamment pour effet d'assurer une protection mécanique du comprimé contre l'effritement ou l'écrasement ou encore de constituer un enrobage entérique ou un enrobage capable de provoquer, après administration du comprimé, un relâchement prolongé du principe actif le contenant.

Habituellement, on choisit cet agent de pelliculage parmi des dérivés de cellulose tels que méthylcellulose, éthylcellulose, propylcellulose ; des dérivés de cellulose hydroxylée tels qu'hydroxyméthylcellulose, hydroxyéthylcellulose, hydroxypropylcellulose, hydroxypropylméthylcellulose ; une cellulose microcristalline ; un polyacrylate tel qu'un copolymère d'acrylate, un ester d'acide acrylique et d'acide méthacrylique, un ester méthylméthacrylique, un polyhydroxyéthyiméthacrytate ; un alcool polyvinylique ; un polyéthylène ; un polypropylène ; un polyéthylèneglycol ; un polystyrène ; un polyacrylamide ; un copolymère éthylvinylacétate polyvinyl acétate ; un copolymère phtalate polyvinyl acétate ; une polyvinylpyrrolidone ; l'oxyde de titane ; une cire.

L'agent de pelliculage en question peut être introduit tel quel dans le fluide supercritique ou de préférence sous forme d'une suspension dans un ou plusieurs solvants appropriés généralement de faible poids moléculaire choisis par exemple parmi des alcools en C₁-C₄ tels que l'éthanol ou l'isopropanol et des esters en C₃-C₆ tels que l'acétate d'éthyle. L'éthanol constitue toutefois un solvant particulièrement préféré.

En outre, les proportions du ou des solvants et d'agent de pelliculage au sein de la suspension seront convenablement choisies de manière à éviter au maximum d'éventuels effets de colmatage du dispositif pour la mise en oeuvre du procédé de l'invention notamment les buses pour l'atomisation de la suspension d'agent de pelliculage au sein du fluide à pression supercritique. Pour cette raison, la quantité d'agent de pelliculage en suspension dans le solvant en question pourra varier de 5% à 30% du poids de la suspension totale en particulier de 10% à 20%.

Selon une variante de cette méthode, la mise en suspension de l'agent de pelliculage au sein du fluide à pression supercritique peut être facilitée par l'addition, à ce fluide, d'un ou de plusieurs co-solvants liquides appropriés. Ce co-solvant également de faible poids moléculaire peut être sélectionné parmi les solvants cités précédemment pour la constitution d'une suspension de l'agent de pelliculage dans un solvant approprié.

Dans cette variante, l'agent de pelliculage peut être introduit tel quel dans le fluide à pression supercritique contenant un ou plusieurs co-solvants ou au contraire sous forme d'une suspension dans un ou plusieurs solvants tel que décrit ci-dessus.

En outre, la quantité d'agent de pelliculage au sein du fluide à pression supercritique n'excédera pas 30% de la masse de celui-ci.

Habituellement, on utilise, dans cette variante un co-solvant identique à celui qui sert à réaliser la suspension de l'agent de pelliculage en particulier l'éthanol.

Le procédé de l'invention ainsi décrit comporte notamment l'avantage d'éviter l'utilisation d'une suspension aqueuse d'agent de pelliculage, ce qui élimine le contact de l'eau avec les comprimés et le séchage ultérieur. De même, on peut ainsi éviter d'utiliser une suspension d'agent de pelliculage dans un solvant organique, ce qui élimine la manipulation de solvants organiques potentiellement toxiques, l'émission importante de composés organiques volatils et le risque de contamination des comprimés par des traces de ces solvants. Selon des mises en oeuvre particulièrement favorables et préférées du procédé faisant l'objet de l'invention, on utilise certaines quantités d'éthanol pour modifier le comportement du fluide à pression supercritique ou pour disperser initialement l'agent de pelliculage afin de rendre sa manipulation plus aisée.

Toutefois, le faible risque de toxicité de l'éthanol n'est absolument pas comparable à celui de la plupart des autres solvants organiques, et les quantités émises de cet alcool dans l'atmosphère peuvent être très limitées selon certaines mises en oeuvre du procédé.

D'autres caractéristiques et avantages du procédé de l'invention apparaîtront au cours de la description suivante en référence aux dessins annexés sur lesquels :
- la Figure 1 représente, de manière schématique, une installation pour le pelliculage de comprimés comprenant un réacteur de préparation de la suspension d'agent de pelliculage dans un fluide à pression supercritique et un tambour de pelliculage, cette installation étant destinée à une mise en oeuvre du procédé de l'invention par voie discontinue.
- la Figure 2 représente, de manière schématique, une installation pour le pelliculage de comprimés destinée à une mise en oeuvre sous pression du procédé de l'invention et par voie discontinue, plus précisément un tambour de pelliculage susceptible d'opérer à pression élevée ainsi qu'un dispositif de recyclage du fluide supercritique.
- la Figure 3 représente de manière schématique une installation pour le pelliculage de comprimés et plus précisément un tambour de pelliculage pour la mise en oeuvre du procédé de l'invention par voie continue.
- la Figure 4 représente de manière schématique une installation pour le pelliculage de comprimés et plus précisément un tambour de pelliculage susceptible d'opérer à pression élevée pour la mise en oeuvre du procédé de l'invention par voie continue.

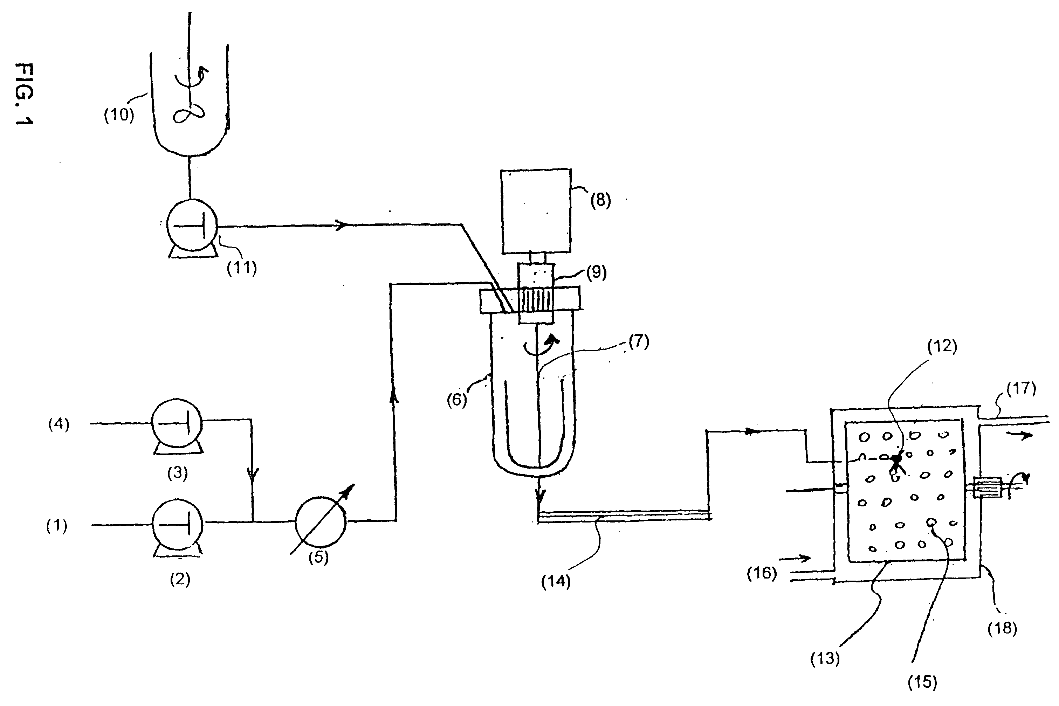

Ainsi, lors d'une mise en oeuvre du procédé de l'invention, par voie discontinue, dans une installation telle que représentée à la Fig 1, le fluide solvant choisi (1), par exemple le dioxyde de carbone, est comprimé par l'intermédiaire d'une pompe volumétrique (2) à membrane et à débit réglable. Grâce à la pompe (3) de même type, dont le débit peut être ajusté, un co-solvant (4) constitué d'un solvant organique liquide, préférentiellement l'éthanol peut être ajouté, si nécessaire, à ce fluide solvant. Le mélange ainsi comprimé à la pression supercritique souhaitée est alors réchauffé à la température choisie, généralement une température voisine de la température critique du fluide solvant et ce, dans un échangeur de chaleur (5) constitué d'un double tube, dont la partie tubulaire extérieure est balayée par de l'eau chaude à température adéquate.

Ce mélange est ensuite introduit dans un réacteur (6) doté d'un agitateur (7) mû par un moteur électrique (8) via un système d'entraînement magnétique (9).

La suspension d'agent de pelliculage (10) sous agitation est également introduite dans le réacteur (6) par l'intermédiaire d'une pompe (11) du même type que les précédentes.

Cette suspension est généralement constituée d'un solvant organique au sein duquel sont dispersés les divers composants de l'agent de pelliculage, ce solvant étant favorablement le même que celui qui est utilisé comme co-solvant au sein du fluide solvant à pression supercritique.

La mise en rotation rapide de l'agitateur (7) permet alors de réaliser une suspension homogène de l'agent de pelliculage dans le mélange solvant porté à pression supercritique.

Cette suspension est alors envoyée vers le tambour de pelliculage (13) au sein duquel elle est décomprimée via une buse d'atomisation (12), la conduite d'amenée de la suspension entre le réacteur (6) et la buse (12) étant réchauffée par un cordon électrique (14) afin d'éviter la précipitation de l'agent de pelliculage et le bouchage de cette conduite. La baisse brutale de la pression entraîne la formation de très fines gouttelettes d'un liquide constitué d'agent de pelliculage dissous ou dispersé au sein du solvant organique. Ces gouttelettes, lorsqu'elles entrent en collision avec les comprimés, les recouvrent d'un film d'autant plus homogène que ces comprimés sont agités en permanence par la mise en rotation du tambour de pelliculage (13). Ce tambour installé au sein d'un récipient (18) est constitué d'un cylindre à axe horizontal dont les parois sont perforées de trous (15) de façon à laisser passer un flux d'air (16) porté à une température de 50 à 60°C.

Le solvant organique est rapidement entraîné par le fluide solvant ramené à pression atmosphérique et par le flux d'air chaud (16) qui permet d'apporter l'enthalpie nécessaire à la vaporisation totale du solvant organique, le gaz formé étant évacué par la conduite (17) reliée à un évent.

Le film d'agent de pelliculage se forme alors de manière uniforme et rapide en raison d'une part de l'élimination complète du solvant organique et du fluide solvant et d'autre part de l'agitation continuelle des comprimés maintenus généralement à la pression atmosphérique ou à une pression voisine, et à la température ambiante, ou à une température voisine, assurant, de cette manière, une bonne répartition de cet agent sur toute la surface de ces comprimés.

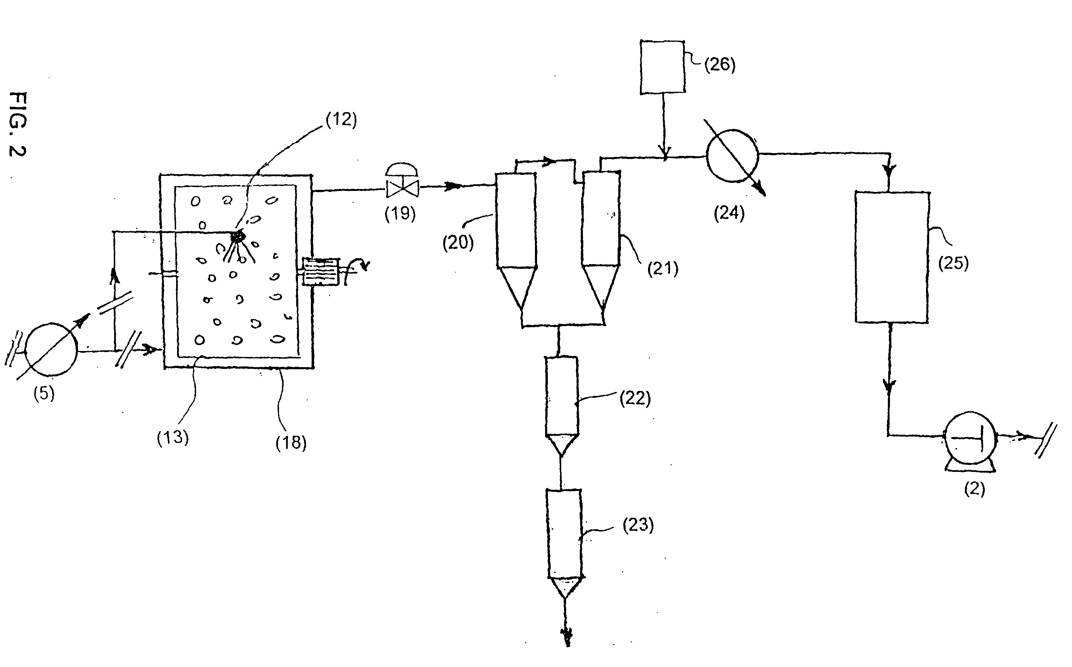

Suivant une variante de la mise en oeuvre précédente par voie discontinue, on peut utiliser une installation telle que représentée à la Fig 1 mais modifiée selon la Fig 2 c'est-à-dire une installation dont le tambour de pelliculage (13) est installé au sein d'un récipient (18) conçu pour supporter une pression d'au moins 200 bars.

Dans cette mise en oeuvre, l'opération de préparation de la suspension d'agent de pelliculage dans le fluide solvant à pression supercritique est conduite dans le même équipement que précédemment de même que la pulvérisation de cette suspension au sein du tambour de pelliculage contenant les comprimés maintenus sous pression généralement à une pression comprise entre 30 et 200 bars par exemple une pression de l'ordre 150 bars et à une température appropriée généralement comprise entre 30 et 60°C par exemple entre 45 et 55°C. Toutefois, on n'y injecte pas d'air chaud comme dans la méthode discontinue ci-dessus mais un flux de fluide solvant (1) porté à une température de 30 à 60°C. Le flux sortant du tambour de pelliculage, constitué d'un mélange de fluide solvant, du co-solvant et du solvant mis en oeuvre pour élaborer la solution d'agent de pelliculage, est ensuite décomprimé, à une pression de l'ordre de 45 bars, via une vanne (19) de type déverseur. Cette décompression entraîne la démixtion du mélange qui est admis dans un ensemble de séparateurs (20) (21) constitués, selon le système décrit dans le brevet FR 2592488 cité précédemment, de chambres cycloniques permettant la séparation totale de la phase liquide et de la phase gazeuse avec apport de chaleur via les parois des séparateurs. A cet effet, la double-enveloppe de ces séparateurs est parcourue par de l'eau chaude, ce qui permet d'apporter l'enthalpie requise pour assurer la vaporisation du fluide solvant. La phase liquide est alors soutirée à pression atmosphérique par l'intermédiaire d'un système de sas (22) (23) fonctionnant selon le système décrit dans le brevet FR 2592488 déjà cité. Le fluide solvant ainsi largement débarrassé des solvant et co-solvant utilisés peut être recyclé. A cet effet, il est liquéfié dans un condenseur double-tube (24) dont la partie tubulaire extérieure est parcourue par un mélange refroidi vers 0°C tel qu'un mélange eau/éthylèneglycol.

Le fluide solvant est ensuite entreposé à l'état liquide vers 5°C dans un réservoir (25) dont le niveau est maintenu de manière stable par appoint de fluide solvant depuis une citerne extérieure (26), avant d'être recyclé par la pompe (2).

Les mises en oeuvre décrites précédemment peuvent être également modifiées de façon à rendre le procédé de l'invention entièrement continu.

En conséquence, un autre objet de l'invention se rapporte à un procédé de pelliculage tel que décrit ci-dessus dans lequel on effectue la pulvérisation de manière continue dans un tambour de pelliculage dont l'axe est incliné par rapport à l'horizontale, par exemple incliné de 5°, permettant une circulation continue des comprimés introduits dans la partie supérieure du tambour et récupérés après pelliculage dans la partie inférieure de ce tambour.

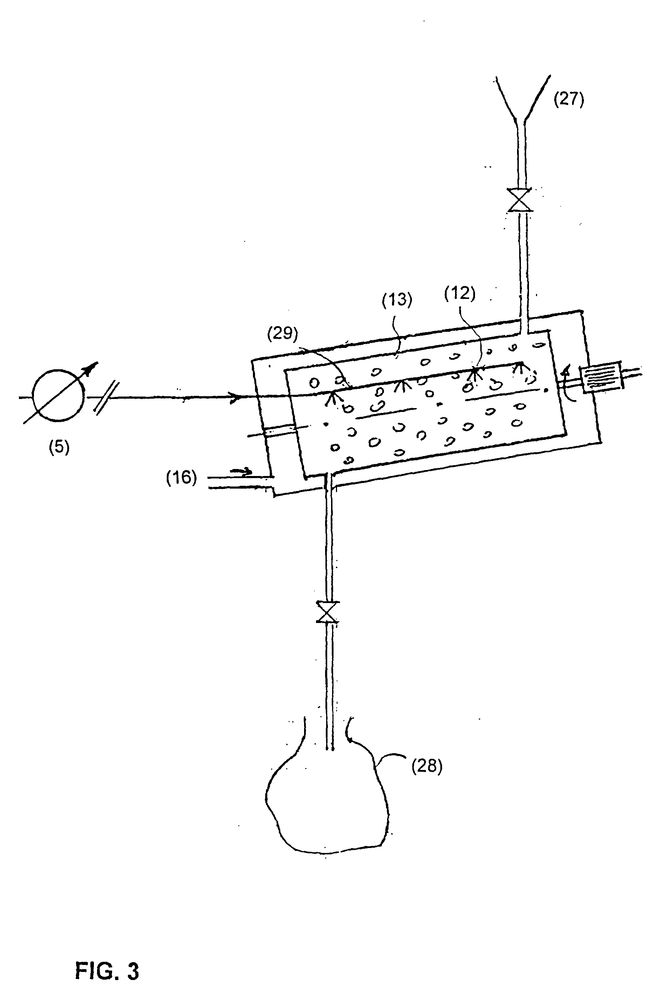

Selon une première mise en oeuvre par voie continue, on peut utiliser une installation telle que représentée à la Fig 1 mais modifiée selon la Fig 3 c'est-à-dire une installation dont le tambour de pelliculage (13) est légèrement incliné par rapport à l'horizontale afin de permettre non seulement la rotation des comprimés mais également leur déplacement longitudinal lent dans le tambour. La longueur et l'obliquité de l'ensemble rotatif (13) sont, en outre, calculés de façon à maintenir un temps de séjour adapté à un pelliculage complet et rapide.

D'autre part, une trémie (27) et un récipient (28) sont connectés l'un à l'entrée située en amont et à la partie supérieure du tambour (13) afin de permettre l'introduction des comprimés de manière semi-continue, l'autre en aval et à sa partie inférieure de manière à récupérer les comprimés pelliculés.

En outre, une rampe (29) de plusieurs buses (12) disposées en parallèle permet la réalisation d'une pulvérisation continue sur les comprimés en mouvement dans le tambour rotatif.

Dans un tel système, il sera possible par modification de la taille des orifices des buses (12) ainsi que par l'obliquité du tambour de modifier le temps de pelliculage complet, selon le résultat visé. En outre le flux gazeux sortant du tambour de pelliculage, est traité par l'un ou l'autre des procédés de récupération/recyclage faisant partie des mises en oeuvre par voie discontinue décrites précédemment.

Selon une variante du procédé par voie continue ci-dessus, on effectue de manière continue la pulvérisation dans un tambour de pelliculage dont l'axe est incliné par rapport à l'horizontale, par exemple incliné de 5°, permettant une circulation continue des comprimés maintenus sous pression dans le tambour, par exemple à une pression comprise entre 30 et 200 bars et à une température appropriée par exemple à une température comprise entre 30 et 60°C, ces comprimés étant introduits dans le tambour de pelliculage grâce à un système de sas situé en amont et à la partie supérieure dudit tambour et récupérés, après pelliculage, en aval grâce à un système de sas situé à la partie inférieure de ce tambour, ces systèmes de sas fonctionnant par voie semi-continue de manière à maintenir une pression dans le tambour en question, par exemple une pression comprise entre 30 et 200 bars.

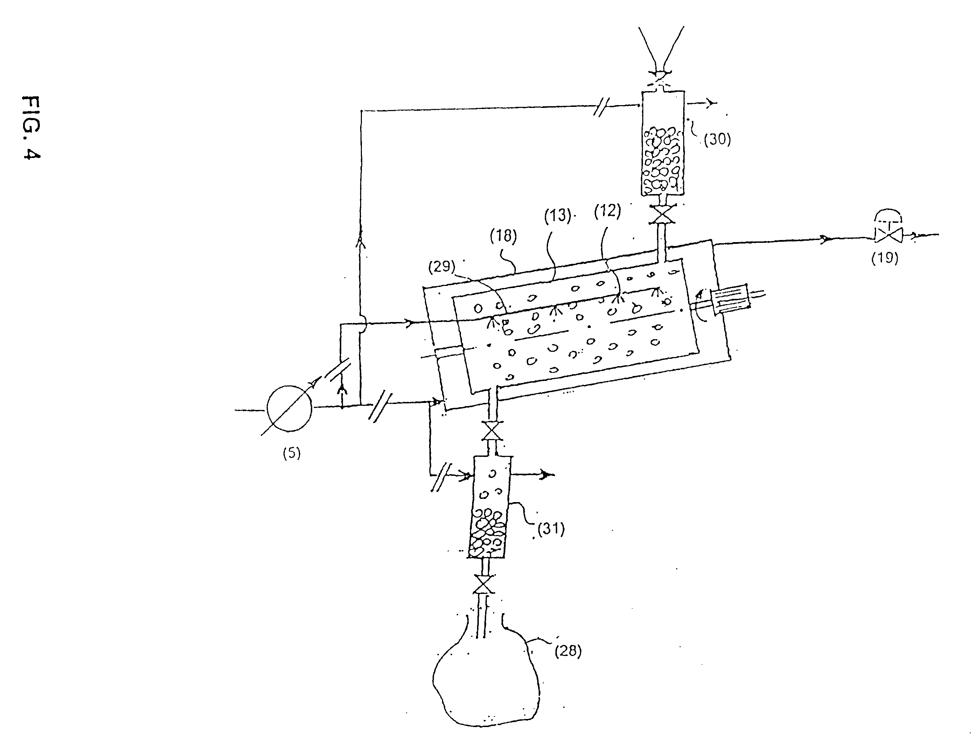

Ainsi, selon une seconde mise en oeuvre par voie continue, on peut utiliser une installation telle que représentée à la Fig 2 mais modifiée selon la Fig 4, c'est-à-dire une installation dont le tambour de pelliculage (13) est placé dans un récipient (18) pouvant résister à une pression de 200 bars.

Son axe, comme celui du récipient sous pression (18) qui le contient, est légèrement incliné par rapport à l'horizontale afin de permettre non seulement la rotation des comprimés mais également leur déplacement longitudinal lent dans le tambour. La longueur et l'obliquité de l'ensemble rotatif sont en outre calculés afin de maintenir un temps de séjour adapté à un pelliculage complet et rapide.

D'autre part, deux sas (30) et (31) sont connectés l'un à l'entrée située à la partie supérieure du tambour (13), l'autre à sa partie inférieure. Ils peuvent être mis sous pression par un courant de fluide solvant dérivé de celui qui assure le balayage du tambour (13). Par ailleurs, une rampe (29) de plusieurs buses (12), disposées en parallèle et selon l'axe du tambour (13), réalise une pulvérisation continue sur les comprimés en mouvement dans le tambour rotatif.

Dans un tel système, il est possible par modification de la taille des orifices des buses ainsi que par l'obliquité du tambour, de modifier le temps de pelliculage total, selon le résultat visé. D'autre part, ce système permet l'introduction des comprimés en continu via le sas (30) et leur récupération selon une quantité analogue via le sas (31) et le récipient (28) en vue par exemple de leur ensachage en ligne. Enfin, comme dans les mises en oeuvre discontinues précédentes, le flux gazeux sortant du tambour de pelliculage peut être traité par les méthodes de récupération/recyclage y décrites.

Les Exemples non limitatifs suivants illustrent le procédé de l'invention.

### EXEMPLE 1

### Pelliculage de comprimés pharmaceutiques contenant l'acide valproïque et son sel de sodium (voie discontinue)

On réalise dans une installation telle qu'illustrée à la Fig 1, le pelliculage d'un lot de comprimés sécables d'une masse unitaire moyenne de 730 mg contenant un principe actif pharmaceutique constitué d'acide valproïque et de son sel de sodium ainsi qu'un excipient constitué de dérivés cellulosiques, de silice et de saccharine sodique avec un agent de pelliculage constitué d'un mélange contenant à parts égales un polyacrylate, un polyéthylène glycol, un dérivé de cellulose de type méthylhydroxypropyl-cellulose, du talc et de l'oxyde de titane, ce pelliculage conduisant à une assimilation retardée du médicament.

Sous agitation pendant 12 heures, on réalise tout d'abord la mise en suspension de l'agent de pelliculage dans de l'éthanol absolu à raison de 250 g de cet agent pour 750g d'éthanol. On introduit ensuite deux litres de cette suspension dans le réacteur (6) et on la met en contact avec du dioxyde de carbone pur maintenu à 150 bars et 45°C. On maintient ce mélange sous agitation au moyen d'un agitateur type ancre de marine tournant à la vitesse de 600 tours par minute.

Après 20 minutes d'agitation, on envoie la suspension ainsi obtenue vers le tambour de pelliculage (13) constitué d'un cylindre à axe horizontal d'un diamètre de 0,48m et d'une longueur de 0,32m dans lequel on a introduit 5 kg de comprimés. On détend brutalement cette suspension jusqu'à la pression atmosphérique à travers une buse (12) dont l'orifice a un diamètre de 350 µm. On met le tambour de pelliculage en rotation à une vitesse de 4 tours par minute et on le balaye par un flux de 100 m³ /h d'air chauffé à 50°C.

Au bout de 5 minutes, après avoir pulvérisé environ 550 g de suspension, on arrête la pulvérisation et on maintient la rotation du tambour et l'envoi d'air chaud pendant 5 minutes afin d'éliminer les dernières traces d'éthanol et de dioxyde de carbone.

Après cette opération, on stoppe le balayage d'air chaud et on arrête la rotation du tambour afin de permettre la récupération des comprimés qui sont ensuite observés à la loupe binoculaire et pesés avec précision. La masse moyenne d'un comprimé pelliculé atteint ainsi 748 mg et la quasi totalité de ces comprimés présente un excellent aspect de surface, analogue à celui obtenu lors de la mise en oeuvre du pelliculage par le procédé classiquement utilisé au départ d'une suspension aqueuse de l'agent de pelliculage. Cette opération de pelliculage selon le procédé antérieur nécessite un temps d'opération de l'ordre de 110minutes sur le même tambour de pelliculage que celui utilisé ci-dessus, soit 10 fois plus de temps qu'en utilisant le procédé faisant l'objet de la présente invention.

### EXEMPLE 2

### Pelliculage de comprimés pharmaceutiques contenant l'acide valproïque et son sel de sodium (voie discontinue)

On réalise dans une installation telle qu'illustrée à la Fig 2, le pelliculage d'un lot de comprimés identiques à ceux utilisés dans l'Exemple 1 avec un agent de pelliculage identique.

Le tambour de pelliculage (13), s'il est semblable à celui utilisé dans l'installation de la Fig 1, est cependant installé dans une enceinte cylindrique (18) conçue pour supporter une pression pouvant atteindre 200 bars, la mise en rotation du tambour étant effectuée via un système à entraînement magnétique assurant une bonne étanchéité à haute pression.

On réalise d'abord, de manière semblable à celle utilisée dans l'Exemple 1, la préparation de la suspension initiale d'agent de pelliculage dans l'éthanol et ensuite la préparation de la suspension au sein du réacteur (6) en présence de dioxyde de carbone à pression supercritique avec toutefois le maintien de la pression au sein de ce réacteur à 190 bars.

Après chargement de 5 kg de comprimés, on referme l'ensemble et on emplit de dioxyde de carbone à 60 bars et 50°C. On maintient un courant de dioxyde de carbone dans ces conditions de pression et de température à raison d'un débit de 100 kg/h, le fluide sortant du tambour étant ensuite détendu à 45 bars environ dans la vanne de détente (19), envoyé vers les séparateurs (20) et (21) et ensuite recyclé comme décrit précédemment.

Lorsque l'équilibre des conditions est bien atteint, on commence la pulvérisation de la suspension réalisée au sein du réacteur (6) dans le tambour de pulvérisation (13) pendant 5 minutes via une buse identique à celle utilisée à l'Exemple 1. Egalement comme dans cet exemple précédent, on maintient le balayage par le dioxyde de carbone et la rotation du tambour (13) pendant cinq minutes après l'arrêt de la pulvérisation, puis on vide le fluide contenu et on ouvre le récipient (18) et le tambour (13) afin de récupérer les comprimés. La pesée et l'observation de ces comprimés donnent des résultats très voisins de ceux obtenus lors de l'Exemple 1 ou de ceux obtenus en mettant en oeuvre un procédé classique, avec une masse moyenne unitaire de 751 mg. Toutefois, à la différence de l'Exemple 1, l'émission d'éthanol à l'atmosphère a été divisée au moins par 10, la plus grande partie de l'éthanol étant récupérée dans les séparateurs (20-21) et soutirée depuis les sas (22-23) à pression atmosphérique. Une faible partie de cet alcool est entraînée par le dioxyde de carbone et est donc immédiatement recyclée dans le procédé. L'émission de solvant dans l'atmosphère est limitée à celle émise lors de la vidange du tambour de pelliculage en fin d'opération, et les émissions de dioxyde de carbone sont elles aussi divisées au moins par 10 par rapport à celles observées lors de l'Exemple 1.

### EXEMPLE 3

### Pelliculage de comprimés pharmaceutiques contenant l'acide valproïque et son sel de sodium (voie continue)

On réalise dans une installation telle qu'illustrée aux Fig 1 et 3, le pelliculage d'un lot de 365kg de comprimés identiques à ceux utilisés dans l'Exemple 1 avec un agent de pelliculage identique.

A cet effet, on prépare, comme décrit à l'Exemple 1, la suspension initiale de cet agent dans l'éthanol, puis la suspension au sein du réacteur (6) en présence de dioxyde de carbone à pression supercritique. De même, on utilise pour la mise en oeuvre de cette opération de pelliculage un équipement analogue à celui utilisé à l'Exemple 1.

Toutefois, le tambour de pelliculage (13) d'un diamètre de 0,48m et d'une longueur de 1,50m est incliné à 5° par rapport à l'horizontale. De plus, l'équipement prévoit la possibilité d'introduire en semi-continu de 2 à 3kg de comprimés moyennant une trémie (27) reliée au tambour (13) par une tuyauterie verticale d'un diamètre de 0,05m connectée à la partie la plus haute du tambour (13) et d'en recueillir dans le récipient (28), toutes les deux minutes environ, une quantité analogue par une tuyauterie verticale de même diamètre que la précédente, connectée dans la partie la plus basse de tambour (13).

Le procédé de pelliculage peut être mis en oeuvre de façon quasi continue le réacteur (6) étant alimenté, d'une part par un débit de suspension d'agent de pelliculage de 10kg/h par la pompe (5) régulé par une sonde de niveau disposée au sein du réacteur (6) et d'autre part en dioxyde de carbone à pression supercritique par la pompe (2) au débit requis pour maintenir une pression de 150 bars dans le réacteur en question.

La pulvérisation est opérée en continu à travers une rampe (29) de quatre buses (12) en parallèle, analogues à celles utilisées dans les exemples précédents, ces buses étant disposées selon l'axe du tambour (13).

On met en oeuvre l'opération de pelliculage de façon analogue à celle décrite à l'Exemple 1 : le tambour de pelliculage est mis en rotation à une vitesse de 4 tours/minute et est balayé par un flux de 100m³/h d'air (16) chauffé à 50°C. Durant une période de 6 heures, on a pu ainsi pelliculer le lot de 365kg de comprimés de façon très satisfaisante, la masse moyenne des comprimés après traitement étant de 752mg.

### EXEMPLE 4

### Pelliculage de comprimés pharmaceutiques contenant l'acide valproïque et son sel de sodium (voie continue)

On réalise dans une installation telle qu'illustrée aux Fig 1 et 4, le pelliculage d'un lot de 345 kg de comprimés identiques à ceux utilisés dans l'Exemple 1 avec un agent de pelliculage identique.

A cet effet, on prépare, comme décrit à l'Exemple 1 la suspension initiale de cet agent dans l'éthanol puis la suspension au sein du réacteur (6) en présence de dioxyde de carbone à pression supercritique.

De même, on utilise pour la mise en oeuvre de cette opération un équipement semblable à celui utilisé à l'Exemple 2. Toutefois, le tambour de pelliculage (13) d'un diamètre de 0,48 m et d'une longueur de 1,50 m, placé dans un récipient (18) qui le contient, est incliné à 5° par rapport à l'horizontale. De plus, deux sas de 10 litres (30) et (31) sont connectés à l'entrée et à la sortie du tambour (13). Ces sas peuvent être mis en pression par un courant de dioxyde de carbone dérivé de celui qui assure le balayage du tambour (13) et permettent d'introduire en semi-continu de 2 à 3 kg de comprimés et d'en extraire une quantité analogue, toutes les deux minutes environ.

Le procédé de pelliculage peut être mis en oeuvre de façon quasi continue, le réacteur (6) étant alimenté d'une part par un débit de suspension de l'agent de pelliculage de 10 kg/h par la pompe (5) régulé par une sonde de niveau disposée au sein du réacteur (6) et d'autre part en dioxyde de carbone supercritique par la pompe (2) au débit requis pour maintenir une pression de 190 bars dans le réacteur en question.

La pulvérisation est opérée en continu à travers une rampe (29) de 4 buses (12) en parallèle, analogues à celles utilisées dans les exemples précédents, ces buses étant disposées selon l'axe du tambour de pelliculage,

On réalise, de façon analogue à celle décrite à l'Exemple 2, la décompression du courant de fluide issu du tambour (13), la séparation et la récupération de l'éthanol via les séparateurs (20) et (21) et les sas (22) et (23) de même que le recyclage du dioxyde de carbone.

Durant une période de 6 heures, on a pu ainsi pelliculer le lot de 345 kg de comprimés de façon très satisfaisante, avec une émission totale d'éthanol de 5,8 kg et une consommation en dioxyde de carbone de 183 kg.

### EXEMPLE 5

### Pelliculage de comprimés contenant le clopidogrel

En utilisant le même procédé que décrit à l'Exemple 2, on a pelliculé un lot de 6 kg de comprimés d'une masse unitaire moyenne de 240 mg comprenant le clopidogrel comme principe actif avec un agent de pelliculage constitué d'un mélange contenant un copolymère phtalate polyvinyl acétate, au départ d'une suspension de 400 g de cet agent de pelliculage dans 1600 g d'éthanol. Cette suspension est mise en contact avec du dioxyde de carbone pur maintenu à l'état supercritique dans des conditions de température et de pression de 50°C et de 150 bars et on procède à l'opération de pelliculage durant 20 minutes.

A l'issue de cette opération, on obtient des comprimés de masse moyenne de 243,6 mg. La quasi totalité de ces comprimés présente un pelliculage satisfaisant.

## Revendications

1. Procédé de pelliculage de comprimés, **caractérisé en ce que** l'on pulvérise sur lesdits comprimés une suspension d'un agent de pelliculage dans un fluide à pression supercritique.

2. Procédé selon la Revendication 1, **caractérisé en ce que** l'on effectue la pulvérisation de manière continue dans un tambour de pelliculage dont l'axe est incliné par rapport à l'horizontale permettant une circulation continue des comprimés introduits en amont et à la partie supérieure de ce tambour et, après pelliculage, récupérés, en aval et à la partie inférieure dudit tambour.

3. Procédé selon la Revendication 1 ou 2, **caractérisé en ce que** l'on effectue la pulvérisation de manière continue dans un tambour de pelliculage dont l'axe est incliné par rapport à l'horizontale permettant une circulation continue des comprimés maintenus à une température appropriée et sous pression dans ledit tambour, ces comprimés étant introduits dans le tambour grâce à un système de sas situé en amont et à la partie supérieure de ce tambour et, après pelliculage, récupérés, en aval grâce à un système de sas situé à la partie inférieure dudit tambour, ces systèmes de sas fonctionnant par voie semi-continue de manière à maintenir une pression dans ledit tambour.

4. Procédé selon une des Revendications 1 à 3, **caractérisé en ce que** la pulvérisation s'opère sur les comprimés maintenus en agitation continue.

5. Procédé selon une des Revendications 1 à 4, **caractérisé en ce que** la suspension d'agent de pelliculage dans le fluide à pression supercritique est formée à partir :
- soit d'un agent de pelliculage introduit tel quel dans le fluide à pression supercritique contenant éventuellement un ou plusieurs co-solvants choisis parmi des alcools en C₁-C₄ et des esters en C₃-C₆
- soit d'une suspension d'un agent de pelliculage dans un ou plusieurs solvants choisis parmi des alcools en C₁-C₄ et des esters en C₃-C₆ introduite dans le fluide à pression supercritique contenant éventuellement un ou plusieurs co-solvants choisis parmi des alcools en C₁-C₄ et des esters en C₃-C₆.

6. Procédé selon la Revendication 5, **caractérisé en ce que** la suspension d'agent de pelliculage dans le ou les solvants varie de 5% à 30% du poids de la suspension totale.

7. Procédé selon la Revendication 5 ou 6, **caractérisé en ce que** le solvant ou co-solvant est l'éthanol.

8. Procédé selon une des Revendications 1 à 7, **caractérisé en ce que** la quantité d'agent de pelliculage dans le fluide à pression supercritique n'excède pas 30% de la masse de celui-ci.

9. Procédé selon une des Revendications 3 à 8, **caractérisé en ce que** la pression est comprise entre 30 et 200 bars.

10. Procédé selon une des Revendications 3 à 9, **caractérisé en ce que** la température est comprise entre 30 et 60°C.

11. Procédé selon une des Revendications 1 à 9, **caractérisé en ce que** l'agent de pelliculage est choisi parmi des dérivés de cellulose, des dérivés de cellulose hydroxylée, une cellulose microcristalline, un polyacrylate, un alcool polyvinylique, un polyéthylène, un polypropylène, un polyéthylèneglycol, un polystyrène, un polyacrylamide, un copolymère éthylvinylacétate polyvinyl acétate, un copolymère phtalate polyvinyl acétate, une polyvinylpyrrolidone, l'oxyde de titane, une cire.

12. Procédé selon une des Revendications 1 à 11, **caractérisé en ce que** le fluide à pression supercritique est le dioxyde de carbone à pression supercritique.

## Patentansprüche

1. Verfahren zur Beschichtung von Tabletten, **dadurch gekennzeichnet, daß** man die Tabletten mit einer Suspension eines Beschichtungsmittels in einem Fluid bei überkritischem Druck besprüht.

2. Verfahren nach Anspruch 1, **dadurch gekennzeichnet, daß** man das Besprühen kontinuierlich in einer Beschichtungstrommel durchführt, deren Achse, bezogen auf die Horizontale, geneigt ist und eine kontinuierliche Zirkulation der Tabletten ermöglicht, die stromaufwärts und im oberen Bereich dieser Trommel eingeführt und nach dem Beschichten stromabwärts und im unteren Bereich der Trommel abgezogen werden.

3. Verfahren nach Anspruch 1 oder 2, **dadurch gekennzeichnet, daß** das Besprühen in kontinuierlicher Weise in einer Beschichtungstrommel bewirkt wird, deren Achse, bezogen auf die Horizontale, geneigt ist und eine kontinuierliche Zirkulation der Tabletten ermöglicht, die bei einer geeigneten Temperatur und unter Druck in der Trommel gehalten werden, wobei diese Tabletten mit Hilfe eines Schleusensystems, welches stromaufwärts und im oberen Bereich dieser Trommel angeordnet ist, in die Trommel eingeführt werden und nach dem Beschichten stromabwärts mit Hilfe eines Schleusensystems, welches im unteren Bereich der Trommel angeordnet ist, aus dem System abgezogen werden, wobei diese Schleusensysteme semikontinuierlich betrieben werden, um einen Druck in der Trommel aufrechtzuerhalten.

4. Verfahren nach einem der Ansprüche 1 bis 3, **dadurch gekennzeichnet, daß** das Besprühen auf die kontinuierlich bewegten Tabletten erfolgt.

5. Verfahren nach einem der Ansprüche 1 bis 4, **dadurch gekennzeichnet, daß** die Suspension des Beschichtungsmittels in dem Fluid bei überkritischem Druck gebildet wird, ausgehend von:
- entweder einem Beschichtungsmittel, welches so, wie es ist, in das Fluid mit überkritischem Druck eingeführt wird, welches gegebenenfalls ein oder mehrere Co-Lösungsmittel ausgewählt aus C₁-C₄-Alkoholen und C₃-C₆-Estern enthält,
- oder einer Suspension eines Beschichtungsmittels in einem oder mehreren Lösungsmitteln ausgewählt aus C₁-C₄-Alkoholen und C₃-C₆-Estern, die in das Fluid mit überkritischem Druck eingeführt wird, welches gegebenenfalls ein oder mehrere Co-Lösungsmittel ausgewählt aus C₁-C₄-Alkoholen und C₃-C₆-Estern enthält.

6. Verfahren nach Anspruch 5, **dadurch gekennzeichnet, daß** die Suspension des Beschichtungsmittels in dem oder den Lösungsmittel(n) von 5 bis 30 Gew.-% der gesamten Suspension variiert.

7. Verfahren nach Anspruch 5 oder 6, **dadurch gekennzeichnet, daß** das Lösungsmittel oder Co-Lösungsmittel Ethanol ist.

8. Verfahren nach einem der Ansprüche 1 bis 7, **dadurch gekennzeichnet, daß** die Menge des Beschichtungsmittels in dem Fluid mit überkritischem Druck 30 Gew.-% der Masse des Fluids nicht übersteigt.

9. Verfahren nach einem der Ansprüche 3 bis 8, **dadurch gekennzeichnet, daß** der Druck zwischen 30 und 200 bar liegt.

10. Verfahren nach einem der Ansprüche 3 bis 9, **dadurch gekennzeichnet, daß** die Temperatur zwischen 30 und 60°C liegt.

11. Verfahren nach einem der Ansprüche 1 bis 9, **dadurch gekennzeichnet, daß** das Beschichtungsmittel ausgewählt ist aus Derivaten von Cellulose, Derivaten von Hydroxycellulose, mikrokristalliner Cellulose, einem Polyacrylat, einem Polyvinylalkohol, einem Polyethylen, einem Polypropylen, einem Polyethylenglykol, einem Polystyrol, einem Polyacrylamid, einem Ethylvinylacetat-Polyvinylacetat-Copolymer, einem Phthalat-Polyvinylacetat-Copolymer. einem Polyvinylpyrrolidon, einem Titanoxid und einem Wachs.

12. Verfahren nach einem der Ansprüche 1 bis 11, **dadurch gekennzeichnet, daß** das Fluid mit überkritischem Druck Kohlendioxid bei überkritischem Druck ist.

## Claims

1. Method for film-coating tablets, **characterized in that** a suspension of a film-coating agent in a fluid at supercritical pressure is sprayed over said tablets.

2. Method according to Claim 1, **characterized in that** the spraying is carried out continuously in a film-coating drum whose axis is inclined relative to the horizontal allowing continuous circulation of the tablets introduced upstream and at the top part of this drum and, after film-coating, recovered, downstream and at the bottom part of said drum.

3. Method according to Claim 1 or 2, **characterized in that** the spraying is carried out continuously in a film-coating drum whose axis is inclined relative to the horizontal allowing continuous circulation of the tablets maintained at an appropriate temperature and under pressure in said drum, these tablets being introduced into the drum by means of a lock chamber system situated upstream and at the top part of this drum and, after film-coating, recovered, downstream by means of a lock chamber system situated at the bottom part of said drum, these lock chamber systems operating semicontinuously so as to maintain a pressure in said drum.

4. Method according to one of Claims 1 to 3, **characterized in that** the spraying is carried out on the tablets kept continuously stirred.

5. Method according to one of Claims 1 to 4, **characterized in that** the suspension of film-coating agent in the fluid at supercritical pressure is formed from:
- either a film-coating agent introduced as it is into the fluid at supercritical pressure optionally containing one or more cosolvents chosen from C₁-C₄ alcohols and C₃-C₆ esters,
- or a suspension of a film-coating agent in one or more solvents chosen from C₁-C₄ alcohols and C₃-C₆ esters introduced into the fluid at supercritical pressure optionally containing one or more cosolvents chosen from C₁-C₄ alcohols and C₃-C₆ esters.

6. Method according to Claim 5, **characterized in that** the suspension of film-coating agent in the solvent(s) varies from 5% to 30% of the weight of the whole suspension.

7. Method according to Claim 5 or 6, **characterized in that** the solvent or cosolvent is ethanol.

8. Method according to one of Claims 1 to 7, **characterized in that** the quantity of film-coating agent in the fluid at supercritical pressure does not exceed 30% of the mass thereof.

9. Method according to one of Claims 3 to 8, **characterized in that** the pressure is between 30 and 200 bar.

10. Method according to one of Claims 3 to 9, **characterized in that** the temperature is between 30 and 60°C.

11. Method according to one of Claims 1 to 9, **characterized in that** the film-coating agent is chosen from cellulose derivatives, hydroxylated cellulose derivatives, a microcrystalline cellulose, a polyacrylate, a polyvinyl alcohol, a polyethylene, a polypropylene, a polyethylene glycol, a polystyrene, a polyacrylamide, an ethylvinyl acetate polyvinyl acetate copolymer, a phthalate polyvinyl acetate copolymer, a polyvinylpyrrolidone, titanium oxide, a wax.

12. Method according to one of Claims 1 to 11, **characterized in that** the fluid at supercritical pressure is carbon dioxide at supercritical pressure.
